# EUROPEAN PATENT SPECIFICATION

(11) **EP 1 707 134 B1**
(45) Date of publication and mention of the grant of the patent: **15.10.2008**
(21) Application number: 06005638.9
(22) Date of filing: 20.03.2006
(51) Int. Cl.: A61B 17/34, A61B 8/08, A61B 10/00, A61B 8/00

(54) **Ultrasonically guided puncturing needle**
Aiguille à ponction guidée ultrasoniquement
Ultraschallgeführte Punktionsnadel

(30) Priority: 30.03.2005 JP 2005099445
(43) Date of publication of application: 04.10.2006
(73) Proprietor: KABUSHIKI KAISHA TOSHIBA, Tokyo 105-8001 (JP); Toshiba Medical Systems Corporation, Otawara-shi, Tochigi-ken 324-8550 (JP)
(72) Inventor: Hiki, Susumu Toshiba Med. Sys. Corp., Otawara-shi Tochigi 324-8550 (JP); Nakaya, Shigemitsu Toshiba Med. Sys. Corp., Otawara-shi Tochigi 324-8550 (JP); Kosaku, Hideki Toshiba Med. Sys. Corp., Otawara-shi Tochigi 324-8550 (JP)
(74) Representative: Kramer - Barske - Schmidtchen

(56) References cited:
- EP-A- 1 426 011
- FR-A- 2 272 633
- US-A- 4 401 124
- US-A- 4 583 061
- US-A- 5 048 530
- US-A- 5 967 988
- US-A- 6 053 870
- US-A1- 2003 135 117

## Description

### ULTRASONICALLY GUIDED PUNCTURING NEEDLE

The present invention relates to an ultrasonically guided puncturing needle that is stabbed in a subject being irradiated with ultrasonic waves for diagnosis or treatment.

What is called ultrasonically guided paracentesis is known in which an operator subjects a lesion site such as tumor which has been found by ultrasonography to puncturing, aspiration biopsy, or cauterization while checking an ultrasonic image of the lesion site. This technique is known to maximize the amount of scattering of ultrasonic waves when the puncturing angle of a needle is set at 60° with respect to an ultrasonic radiation angle. Thus, when the puncturing angle of the needle is not 60°, the amount of backscattering of ultrasonic waves at the tip of the needle may decrease to prevent the ultrasonic waves from being appropriately received. To obtain clear needle tip echoes, it is thus necessary to set the puncturing angle of the needle as close to 60° as possible.

Thus, when this technique is used, a puncturing guide is used which guides the direction in which the needle is inserted. The puncturing guide is commonly fixed to an ultrasonic probe to set the puncturing angle of the needle at 60° with respect to the ultrasonic irradiation angle.

However, even though the inserting direction of the needle is guided using the puncturing guide, the needle itself may be bent during the puncturing process to prevent the puncturing angle from being maintained at 60° near the lesion site. In other cases, another angle may have to be chosen depending on the positional relationship between the ultrasonic probe and the lesion. In the above case, the amount of backscattering of the ultrasonic wave at the needle tip may decrease to make needle tip echoes unclear.

FR-A-2272633 discloses a device as described in the preambule of claim 1 and US 6053870 discloses a device as described in the preambule of claim 3.

In recent years, a technique relating to a film has been developed in which a gas is used as a reflection source for ultrasonic waves in order to obtain clear needle tip echoes. The gas provides an acoustic impedance significantly different from that of living bodies and can thus be very effectively used as a reflection source for ultrasonic waves (see, for example, PCT National Publication No. 2001-504101).

However, a problem with the technique described in PCT National Publication No. 2001-504101 is that manufacture of the film is very complicated, thus requiring high manufacture costs.

The present invention provides an ultrasonically guided puncturing needle as defined in claim 1 that enables a safe, reliable technique for ultrasonically guided paracentesis to be realized without the need for special equipment or control. Further embodiments are defined in the sub-claims. are formed.
(2) The ultrasonically guided puncturing needle set forth in (1), wherein a space which is either a gas layer or a vacuum layer is formed in each of the concaves.
(3) The ultrasonically guided puncturing needle set forth in (2), wherein the concaves and convexes are formed on an outer peripheral surface of the needle-like member, and a distance from an outer surface of the film formed on the outer peripheral surface to the space is equal to or shorter than the wavelength of the ultrasonic wave.
(4) An ultrasonically guided puncturing needle stabbed in a subject being irradiated with an ultrasonic wave, the needle comprising a cylindrical needle-like member having a plurality of holes in a peripheral wall and a film which blocks the plurality of the holes.
(5) The ultrasonically guided puncturing needle set forth in (4), wherein a space which is either a gas layer or a vacuum layer is formed in each of the holes.
(6) The ultrasonically guided puncturing needle set forth in (4), wherein the film is formed on an outer peripheral surface of the needle-like member, and
   a distance from an outer surface of the film to the space is equal to or shorter than the wavelength of the ultrasonic wave.
(7) The ultrasonically guided puncturing needle set forth in (4), wherein the film blocks the plurality of the holes from an outside of the needle-like member.
(8) The ultrasonically guided puncturing needle set forth in (4), wherein the film blocks the plurality of the holes from an inside of the needle-like member.
(9) An ultrasonically guided puncturing needle stabbed in a subject being irradiated with an ultrasonic wave, the needle comprising a cylindrical needle-like member having a plurality of concaves on an

The invention can be more fully understood from the following detailed description when taken in conjunction with the accompanying drawings, in which:
FIG. 1 is a schematic diagram showing a use environment for an ultrasonically guided puncturing needle according to a first embodiment of the present invention;
FIG. 2A is a schematic diagram of the ultrasonically guided puncturing needle according to the first embodiment;
FIG. 2B is a sectional view of the ultrasonically guided puncturing needle according to the first embodiment;
FIG. 3 is a conceptual drawing showing that an ultrasonic wave is reflected by an air layer according to the first embodiment;
FIG. 4A is a schematic diagram of an ultrasonically guided puncturing needle according to a second embodiment of the present invention;
FIG. 4B is a sectional view of the ultrasonically guided puncturing needle according to the second embodiment of the present invention;
FIG. 5A is a schematic diagram of the ultrasonically guided puncturing needle according to a third embodiment of the present invention;
FIG. 5B is a sectional view of the ultrasonically guided puncturing needle according to the third embodiment of the present invention;
FIG. 6A is a process diagram showing a process of manufacturing an ultrasonically guided puncturing needle according to the third embodiment;
FIG. 6B is a process diagram showing the process of manufacturing an ultrasonically guided puncturing needle according to the third embodiment; and
FIG. 6C is a process diagram showing the process of manufacturing an ultrasonically guided puncturing needle according to the third embodiment.

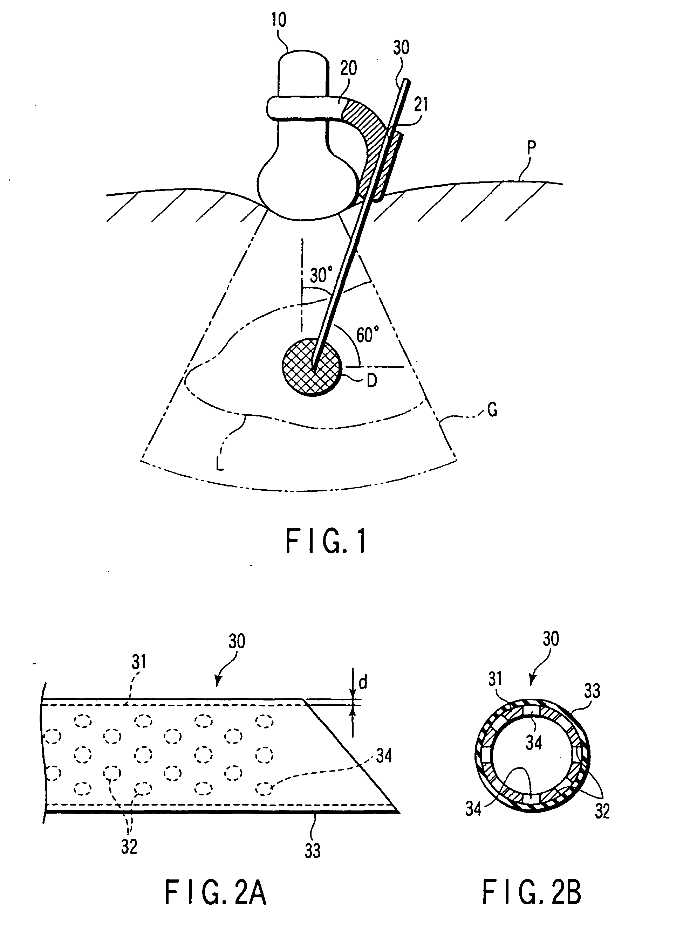

First, a use environment for an ultrasonically guided puncturing needle 30 will be described. FIG. 1 is a schematic diagram showing the use environment for the ultrasonically guided puncturing needle 30 In FIG. 1, reference numerals 10, 20, and 30 denote an ultrasonic probe, a puncturing guide, and the ultrasonically guided puncturing needle, respectively. Reference characters P and G denote a subject and an ultrasonic image.

The ultrasonic probe 10 transmits and receives ultrasonic waves through a transmitting and receiving surface provided at a leading end of the ultrasonic probe 10 to the subject P in order to visualize the internal structure of the subject P. An ultrasonic image G of the subject P is displayed on a monitor (not shown). Here, the ultrasonic image G is drawn on the subject P in FIG. 1.

The puncturing guide 20 is fixed to the ultrasonic probe 10 and has a guide hole 21 formed at a predetermined position. The ultrasonically guided puncturing needle 30 is inserted through the guide hole 21 so as to be movable forward and backward. The ultrasonically guided puncturing needle 30 is guided so as to have a fixed puncturing angle. The puncturing angle of the ultrasonically guided puncturing needle 30 is set at about 60°. That is, the ultrasonically guided puncturing needle 30 is stabbed while being inclined at about 30° to the axis of the ultrasonic probe 10 in an array direction. The ultrasonically guided puncturing needle 30 is not inclined in a lens direction.

The ultrasonically guided puncturing needle 30 sucks or cauterizes a biotissue in a lesion site D or inject alcohol into the lesion site D, via its leading end. In the present embodiment, the lesion site D is assumed to be a cancer in the liver L.

### (Configuration of the Ultrasonically Guided Puncturing Needle 30)

Now, the configuration of the ultrasonically guided puncturing needle 30 will be described with reference to FIGS. 2A and 2B. FIG. 2A is a schematic diagram of the ultrasonically guided puncturing needle 30 . FIG. 2B is a sectional view of the ultrasonically guided puncturing needle 30.

As shown in FIGS. 2A and 2B, the ultrasonically guided puncturing needle 30 comprises a needle main body (needle-like member) 31. The needle main body 31 is formed to be cylindrical and its leading end stabbed in the subject P is reverse-tapered so as to prevent the biotissue from being caught. A metal material is used for the needle main body 31.

A large number of holes 32 are formed in a peripheral wall of the needle main body 31 to allow the inside and outside of the needle main body 31 to communicate with each other. The shape of the hole 32 is not limited but the pitch intervals of the holes are preferably as small as possible. For example, laser machining is used to form holes 32.

A film 33 is formed around an outer peripheral surface of the needle main body 31. The film 33 has a film thickness d equal to or smaller than the wavelength of an ultrasonic wave. The film 33 externally blocks the large number of holes 32 formed in the needle main body 31. This forms a plurality of air layers 34 in the needle main body 31 which are accessible to ultrasonic waves. In order to prevent the needle from being markedly hindered from being inserted into the living body owing to the presence of the holes 32 formed in the needle main body 31, the film 33 is preferably made of resin, which allows a film to be appropriately formed around the needle main body 31 and which is safe for living bodies. The air layers 34 are necessarily formed by the reduced adhesion at the boundary between the hole 32 and the film 33 resulting from the formation of a film 33.

Now, the usage of the ultrasonically guided puncturing needle 30 will be described. The operator applies the transmitting and receiving surface of the ultrasonic probe 10 to the subject P and starts transmitting and receiving an ultrasonic wave. This causes an ultrasonic image G of a region including the lesion site D to be displayed on the monitor (not shown).

The operator then inserts the ultrasonically guided puncturing needle 30 into the guide hole 21 in the puncturing guide 20. While viewing the ultrasonic image G, the operator stabs the ultrasonically guided puncturing needle 30 in the subject P. The ultrasonically guided puncturing needle 30 stabbed in the subject P is shown in the ultrasonic image G as shown in FIG. 1. Accordingly, while viewing the ultrasonically guided puncturing needle 30 shown in the ultrasonic image G, the operator aligns the leading end of the ultrasonically guided puncturing needle 30 with the lesion site D. The operator then performs an operation such as sucking or cauterization of a biotissue in the lesion site D, injection of alcohol into the lesion site D, or the like. After the operation, the operator removes the ultrasonically guided puncturing needle 30 from the subject P while viewing the ultrasonic image G. The ultrasonically guided paracetesis is thus finished.

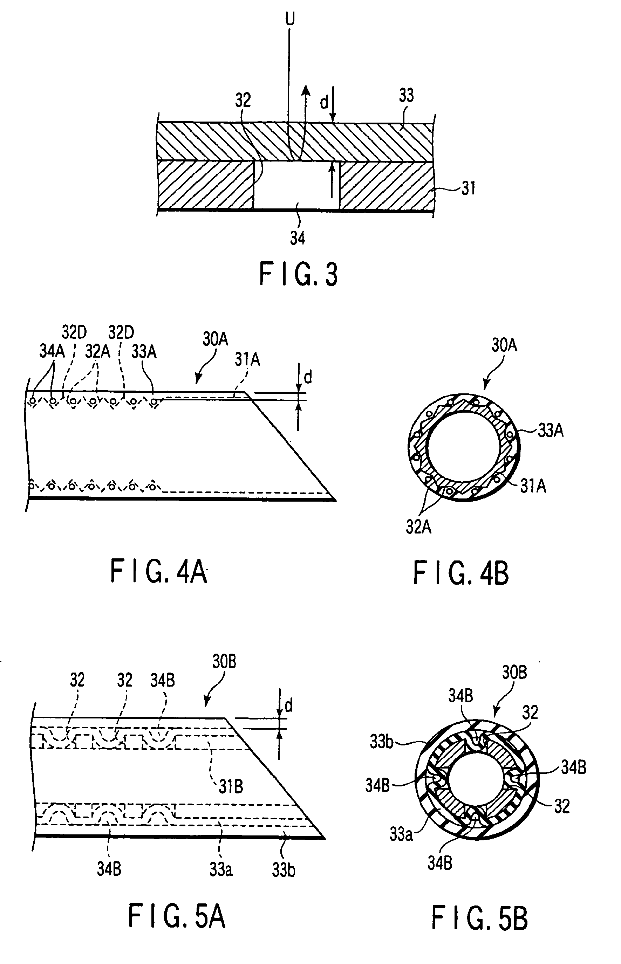

Now, display of the ultrasonically guided puncturing needle 30 will be described with reference to FIG. 3. FIG. 3 is a conceptual drawing showing that an ultrasonic wave is reflected by the air layer 34 Ultrasonic waves transmitted by the ultrasonic probe 10 pass through a tissue in the subject P to reach the ultrasonically guided puncturing needle 30. An ultrasonic wave U which reached a portion of the film 33 corresponding to the hole 32 is transmitted through the film 33 and reflected by the boundary surface between the film 33 and the air layer 34 as shown in FIG. 3. An ultrasonic wave which reached a portion of the film 33 corresponding to the needle main body 31 is transmitted through the film 33 and reflected by the boundary surface between the film 33 and the needle main body 31. The ultrasonic wave reflected by the air layer 34 or needle main body 31 is transmitted through the film 33 and the tissue in the subject P again and then received by the ultrasonic probe 10.

The air layer 34 and the subject P have greatly different acoustic impedances. The ultrasonic wave reflected by the air layer 34 thus has a very large intensity. Consequently, if the ultrasonically guided puncturing needle 30 comprises the large number of air layers 34 , the amount of backscattering at the tip of the ultrasonically guided puncturing needle 30 increases to brightly show the ultrasonically guided puncturing needle 30 on the ultrasonic image G.

The large number of holes 32 are formed in the peripheral wall of the needle main body 31. The air layers 34 are also provided in the needle main body 31 by blocking the holes 32 from the outside of the needle main body 31 with the film 33.

This increases the amount of backscattering at the tip of the ultrasonically guided puncturing needle 30. The ultrasonically guided puncturing needle 30 is thus brightly shown even if the puncturing angle of the ultrasonically guided puncturing needle 30 is markedly different from 60°. Safe, reliable operations can also be performed without the need for special equipment or control.

Moreover, the present device only requires the formation of a large number of holes 32 in the needle main body 31 and the formation of a film 33 around the outer peripheral surface of the needle main body 31. The ultrasonically guided puncturing needle can be obtained by a very simple manufacture process.

The present device has been described in conjunction with the puncturing angle in the array direction. Even if, for example, the ultrasonically guided puncturing needle 30 is greatly bent in the lens direction during the puncturing process, the amount of backscattering at the tip of the ultrasonically guided puncturing needle 30 increases to enable the ultrasonically guided puncturing needle 30 to be shown more brightly than in the prior art.

First, the configuration of an ultrasonically guided puncturing needle 30A will be described with reference to FIGS. 4A and 4B. FIG. 4A is a schematic diagram of the ultrasonically guided puncturing needle 30A. FIG. 4B is a sectional view of the ultrasonically guided puncturing needle 30A according to the second embodiment.

As shown in FIGS. 4A and 4B, the ultrasonically guided puncturing needle 30A comprises a large number of concaves 32A and convexes 32D in an outer peripheral surface of a needle main body (needle-like member) 31A. The shape of the concave 32A and convex 32D is not limited but the pitch intervals are preferably as small as possible. The concaves 32A and the convexes 32D are formed by, for example, sand blasting. Concaves and convexes on an inner peripheral surface can be formed by rotationally inserting a screw-like machine having an outer diameter equal to the inner diameter of the needle main body 31A into the needle main body 31A.

A film 33A is formed around the outer peripheral surface of the needle main body 31A. The film 33A externally blocks the large number of concaves 32A formed in the outer peripheral surface of the needle main body 31A. A small void is formed inside each concave 32A. The distance d from the surface of the film 33A to the void is set equal to or shorter than the wavelength of ultrasonic waves when by conditions are set for the formation of a film 33A. This forms a large number of air layers 34A in the concaves 32A which consist of the voids and which are reachable by supersonic waves.

In the present device, the large number of concaves 32A are formed around the outer peripheral surface of the needle main body 31A. The air layers 34A are provided in the needle main body 31A by blocking the large number of concaves 32A from the outside of the needle main body 31A with the film 33A.

This increases the amount of backscattering at the tip of the ultrasonically guided puncturing needle 30A. The ultrasonically guided puncturing needle 30A is thus brightly shown even if the puncturing angle of the ultrasonically guided puncturing needle 30A is markedly different from 60°. Safe, reliable operations can also be performed without the need for special equipment or control. Moreover, the ultrasonically guided puncturing needle 30A can be obtained by a very simple manufacture process.

The present device uses the air layers 34A to increase the amount of backscattering at the tip of the ultrasonically guided puncturing needle 30A. However, the present invention is not limited to this. Any layer, for example, a vacuum layer, may be used provided that it reflects ultrasonic waves well. The vacuum layer is easily obtained provided that a film 33A is formed around the needle main body 31A in a vacuum environment.

First, the configuration of an ultrasonically guided puncturing needle 30B will be described with reference to FIGS. 5A and 5B. FIG. 5A is a schematic diagram of the ultrasonically guided puncturing needle 30B FIG. 5B is a sectional view of the ultrasonically guided puncturing needle 30B

As shown in FIGS. 5A and 58, the ultrasonically guided puncturing needle 30B comprises the large number of holes 32 in an outer peripheral surface of a needle main body 31B.

A first and second films 33a and 33b are sequentially stacked around the outer peripheral surface of the needle main body 31B. The first film 33a gets into the holes 32, formed in the needle main body 31B, and has concaves formed in its outer peripheral surface at positions corresponding to the holes 32. The second film 33b has a film thickness d equal to or shorter than the wavelength of ultrasonic waves and almost completely cylindrical; the shape of the second film 33b does not coincide with the outer peripheral surface of the first film 33a. This forms a large number of air layers 34B outside the needle main body 31B at positions corresponding to the holes 32; the air layers 34B are blocked by the first and second film 33a and 33b.

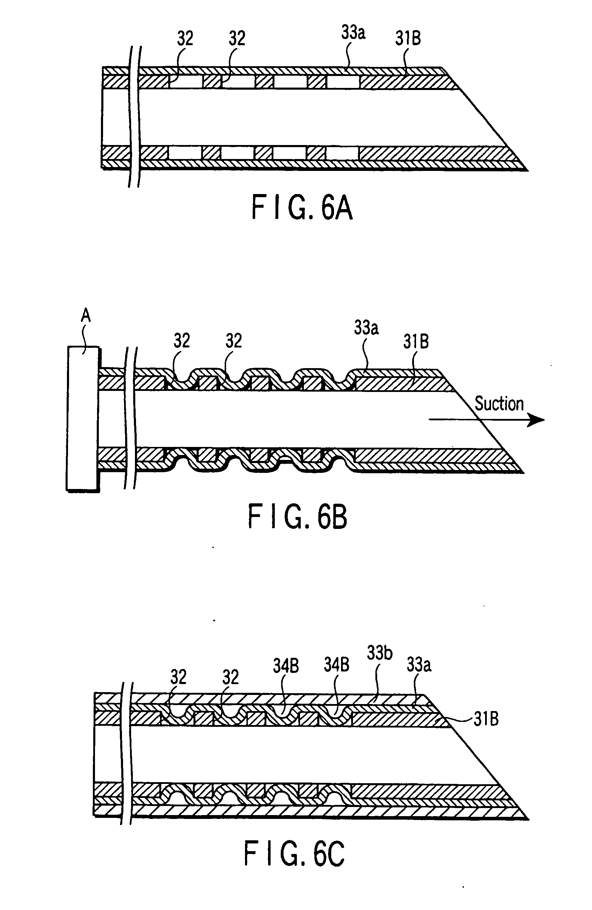

Now, with reference to FIGS. 6A to 6C, description will be given of a process of manufacturing an ultrasonically guided puncturing needle 30B. FIGS. 6A to 6C is a process diagram showing the process of manufacturing an ultrasonically guided puncturing needle 30 30B.

As shown in FIG. 6A, a first film 33a is formed around the outer peripheral surface of the needle main body 31B. Then, as shown in FIG. 6B, a base end of the needle main body 31B is closed by a closing member A. Air is sucked from the needle main body 31B through a leading end of the needle main body 31B. This causes the first film 33a to be sucked into the holes 32 to form concaves in the outer peripheral surface of the first film 33a. Then, as shown in FIG. 6C, a second film 33b is formed around the outer peripheral surface of the first film 33a. This forms a large number of air layers 34B around the outer peripheral surface of the needle main body 31B at positions corresponding to the holes 32; the air layers 34B are blocked by the first and second films 33a and 33b.

In the present device, the large number of holes 32 are formed in the peripheral wall of the needle main body 31B. The first and second films 33a and 33b are stacked on the outer peripheral surface of the needle main body 31B. The air layers 34B are provided between the first and second films 33a and 33b to reflect ultrasonic waves.

This increases the amount of backscattering at the tip of the ultrasonically guided puncturing needle 30B. The ultrasonically guided puncturing needle 30B is thus brightly shown even if the puncturing angle of the ultrasonically guided puncturing needle 30A is markedly different from 60°. Safe, reliable operations can also be performed without the need for special equipment or control. Moreover, the ultrasonically guided puncturing needle 30B according to the present invention can be obtained by a very simple manufacture process.

The present device uses the air layers 34B to increase the amount of backscattering at the tip of the ultrasonically guided puncturing needle 30B. However, the present invention is not limited to this. Any layer, for example, a vacuum layer, may be used provided that it reflects ultrasonic waves well. The vacuum layer is easily obtained provided that a second film 33b is formed around the needle main body 31B in a vacuum environment.

Various inventions can also be formed by appropriately combining a plurality of the components disclosed in the above devices. For example, some of the components shown in the devices may be deleted. Components of different devices may also be appropriately combined together.

It is explicitly stated that all features disclosed in the description and/or the claims are intended to be disclosed separately and independently from each other for the purpose of original disclosure as well as for the purpose of restricting the claimed invention independent of the composition of the features in the embodiments and/or the claims. It is explicitly stated that all value ranges or indications of groups of entities disclose every possible intermediate value or intermediate entity for the purpose of original disclosure as well as for the purpose of restricting the claimed invention, in particular as limits of value ranges.

## Claims

1. An ultrasonically guided puncturing needle (30A) for stabbing into a subject (P) being irradiated with an ultrasonic wave, the needle (30A) comprising:
a cylindrical needle-like member (31A) having concaves (32A) and convexes (32D) formed on an outer peripheral surface of the needle-like member (31A) to reflect the ultrasonic wave;
**characterized in that** the needle further comprises a film (33A) formed on the peripheral surface on which the concaves (32A) and convexes (32D) are formed.
a space which is either a gas layer (34A) or a vacuum layer is formed in each of the concaves (32A).

2. The ultrasonically guided puncturing needle (30A) according to claim 1, wherein a distance from an outer surface of the film (33A) formed on the outer peripheral surface to the space is equal to or shorter than the wavelength of the ultrasonic wave.

3. An ultrasonically guided puncturing needle (30) for stabbing into a subject (P) being irradiated with an ultrasonic wave, the needle (30) comprising:
a cylindrical needle-like member (31) having a plurality of holes (32) in a peripheral wall;
**characterized by**
a film (33; 33a) which blocks the plurality of the holes (32),
in that a space which is either a gas layer (34) or a vacuum layer is formed in each of the holes (32), and
in that the film (33; 33a) blocks the plurality of the holes (32) from an outside of the needle-like member (31).

4. The ultrasonically guided puncturing needle (30) according to claim 3, **characterized in that** the film (33) is formed on an outer peripheral surface of the needle-like member (31), and
a distance from an outer surface of the film (33) to the space is equal to or shorter than the wavelength of the ultrasonic wave.

5. The ultrasonically guided puncturing needle according to claim 3, further comprising
at least a second film (33b) such that at least two films (33a, 33b) are stacked on the peripheral surface of the needle-like member (31B),
wherein the space which is either a vacuum layer or a gas layer (34B) is formed between the two films (33a, 33b).

6. The ultrasonically guided puncturing needle (30B) according to claim 5, **characterized in that** a distance from an outer surface of the outermost one (33b) of the at least two films (33a, 33b) to the space is equal to or shorter than the wavelength of the ultrasonic wave.

## Patentansprüche

1. Ultraschallgeführte Punktionsnadel (30A) zum Stechen in ein Objekt (P), das mit einer Ultraschallwelle bestrahlt wird, wobei die Nadel (30A) aufweist
ein zylindrisches nadelähnliches Element (31A) mit Konkaven (32A) und Konvexen (32D), die auf einer äußeren Umfangsfläche des nadelähnlichen Elements (31A) gebildet sind, um die Ultraschallwelle zu reflektieren; **dadurch gekennzeichnet, dass**
die Nadel ferner einen Film (33A) aufweist, der auf der Umfangsfläche, auf der die Konkaven (32A) und Konvexen (32D) gebildet sind, gebildet ist; und
ein Raum, der entweder eine Gasschicht (34A) oder eine Vakuumschicht ist, in jedem der Konkaven (32A) gebildet ist.

2. Ultraschallgeführte Punktionsnadel (30A) nach Anspruch 1, wobei eine Distanz von einer äußeren Fläche des Films (33A), der auf der äußeren Umfangsfläche gebildet ist, zu dem Raum gleich oder kürzer als die Wellenlänge der Ultraschallwelle ist.

3. Ultraschallgeführte Punktionsnadel (30) zum Stechen in ein Objekt (P), das mit einer Ultraschallwelle bestrahlt wird, wobei die Nadel (30) aufweist
ein zylindrisches nadelähnliches Element (31) mit einer Mehrzahl von Löchern (32) in einer Umfangswand;
**gekennzeichnet durch**
einen Film (33; 33a), der die Mehrzahl der Löcher (32) blockiert, wobei
ein Raum, der entweder eine Gasschicht (34) oder eine Vakuumschicht ist, in jedem der Löcher (32) gebildet ist, und
der Film (33; 33a) die Mehrzahl der Löcher (32) von außerhalb des nadelähnlichen Elements (31) blockiert.

4. Ultraschallgeführte Punktionsnadel (30) nach Anspruch 3, **dadurch gekennzeichnet, dass** der Film (33) auf einer äußeren Umfangsfläche des nadelähnlichen Elements (31) gebildet ist, und
eine Distanz von einer äußeren Fläche des Films (33) zu dem Raum gleich oder kürzer als die Wellenlänge der Ultraschallwelle ist.

5. Ultraschallgeführte Punktionsnadel nach Anspruch 3, ferner mit
mindestens einem zweiten Film (33b), so dass mindestens zwei Filme (33a, 33b) auf der Umfangsfläche des nadelähnlichen Elements (31 B) gestapelt sind,
wobei der Raum, der entweder eine Vakuumschicht oder eine Gasschicht (34B) ist, zwischen den zwei Filmen (33a, 33b) gebildet ist.

6. Ultraschallgeführte Punktionsnadel (30B) nach Anspruch 5, **dadurch gekennzeichnet, dass** eine Distanz von einer äußeren Fläche des äußersten Films (33b) von den mindestens zwei Filmen (33a, 33b) zu dem Raum gleich oder kürzer als die Wellenlänge der Ultraschallwelle ist.

## Revendications

1. Aiguille à ponction guidée par ultrasons (30A) destinée à piquer un sujet irradié par une onde ultrasonique, l'aiguille (30A) comprenant :
un élément cylindrique de type aiguille (31A) ayant des formes concaves (32A) et des formes convexes (32D) ménagées sur une surface périphérique externe de l'élément de type aiguille (31A) pour réfléchir l'onde ultrasonique ;
**caractérisée en ce que**
l'aiguille comprend en outre un film (33A) formé sur la surface périphérique sur laquelle les formes concaves (32A) et les formes convexes (32D) sont ménagées, et
un espace qui est soit une couche de gaz (34A), soit une couche de vide est formé dans chacune des formes concaves (32A).

2. Aiguille à ponction guidée par ultrasons (30A) selon la revendication 1, dans laquelle la distance entre une surface externe du film (33A) formé sur la surface périphérique externe et l'espace est égale ou inférieure à la longueur d'onde de l'onde ultrasonique.

3. Aiguille à ponction guidée par ultrasons (30) destinée à piquer un sujet (P) irradié par une onde ultrasonique, l'aiguille (30) comprenant :
un élément cylindrique de type aiguille (31) ayant une pluralité de trous (32) dans une paroi périphérique ;
**caractérisée en ce que** :
elle présente un film (33 ; 33a) qui bloque la pluralité des trous (32),
un espace qui est soit une couche de gaz (34), soit une couche de vide, est formé dans chacun des trous (32), et
le film (33 ; 33a) bloque la pluralité des trous (32) de l'extérieur de l'élément de type aiguille (31).

4. Aiguille à ponction guidée par ultrasons (30) selon la revendication 3, **caractérisée en ce que** le film (33) est formé sur une surface périphérique externe de l'élément de type aiguille (31), et une distance entre une surface externe du film (33) et l'espace est égale ou inférieure à la longueur d'onde de l'onde ultrasonique.

5. Aiguille à ponction guidée par ultrasons selon la revendication 3, comprenant en outre :
au moins un second film (33b) tel qu'au moins deux films (33a, 33b) soient empilés sur la surface périphérique de l'élément de type aiguille (31B),
dans laquelle l'espace qui est soit une couche de vide, soit une couche de gaz (34B) est formé entre les deux films (33a, 33b).

6. Aiguille à ponction guidée par ultrasons (30B) selon la revendication 5, **caractérisée en ce qu'**une distance entre une surface externe d'un film le plus à l'extérieur (33b) parmi les au moins deux films (33a, 33b) et l'espace est égale ou inférieure à la longueur d'onde de l'onde ultrasonique.
